# EUROPEAN PATENT APPLICATION

(11) **EP 1 568 372 A1**
(43) Date of publication of application: **31.08.2005**
(21) Application number: 04290530.7
(22) Date of filing: 26.02.2004
(51) Int. Cl.: A61K 31/727, A61P 7/02, A61P 9/10

(54) **Administration of enoxaparin sodium to patients with severe renal impairment**

(71) Applicant: Aventis Pharma S.A., 92160 Antony (FR)
(72) Inventor: The designation of the inventor has not yet been filed
(74) Representative: Le Pennec, Magali

(57) **Abstract**

Methods having novel enoxaparin sodium dosing regimens for patients with severe renal impairment are disclosed. The methods may be used for one or more of preventing thrombotic episodes, treating thrombotic episodes, preventing postoperative venous thrombosis, controlling thrombosis and/or decreasing blood hypercoagulation and/or hemorrhaging risks, treating unstable angina, and treating non-Q-wave myocardial infarction in human patients with severe renal impairment. The methods of preventing thrombotic episodes, treating t hrombotic episodes, preventing postoperative venous thrombosis, and controling thrombosis and/or decreasing blood hypercoagulation and/or hemorrhaging risks, comprise administering from more than 20 mg to less than 40 mg, from 25 mg to 35 mg, about 30 mg, or 30 mg of enoxaparin sodium to the patient once daily. The methods of treating unstable angina, and non-Q-wave myocardial infarction comprise administering from more than 0.5 mg/kg body weight to less than 1.5 mg/kg body weight, or about 1 mg/kg body weight of enoxaparin sodium once daily. Articles of manufacture comprising enoxaparin sodium and instructions for the use of the enoxaprin sodium are also disclosed.

## Description

Broadly, this invention is directed to novel dosing regimens for administering enoxaparin sodium (sometimes referred to hereinafter as "enoxaparin") to patients with severe renal impairment. The invention also relates to methods of using such dosing regimens of enoxaparin for one or more of preventing thrombotic episodes, treating thrombotic episodes, preventing postoperative venous thrombosis, controlling thrombosis and/or decreasing blood hypercoagulation and/or hemorrhaging risks, treating unstable angina, and treating non-Q-wave myocardial infarction, in human patients with severe renal impairment (creatinine clearance < 30 mL/min), defined herein. This invention also provides articles of manufacture for use in connection with a patient with severe renal impairment, comprising enoxaparin and instructions designed to achieve administration of the enoxaparin.

Enoxaparin sodium is available from Aventis under the trademark Lovenox® (Clexane® in some other countries). Enoxaparin sodium exhibits renal clearance. Study data have demonstrated a significant impact of renal function on enoxaparin pharmacokinetics as measured by plasma anti-Xa activity. The main effect of renal impairment is a reduced clearance, which results in a significantly longer elimination half-life. This in turn results in higher pre-dose levels after repeated administration and then an increase in Amax (maximum observed activity) after repeated dosing.

Elevated levels of enoxaparin sodium in patients with severe renal impairment may cause undesirable effects, such as bleeding resulting from excessive anticoagulation. The possibility of a dose adjustment in patients with severe renal impairment has been suggested. A difficulty, however, in determining the appropriate amount of any adjustment in patients with severe renal impairment results from the need to balance the desired antithrombotic activity of enoxaparin sodium against the possibility of excessive bleeding or other undesired results of enoxaparin sodium accumulation.

As used herein, "severe renal impairment" means an impairment of renal function in a patient characterized by a creatinine clearance rate of < 30 mL/min.

The term "prevent," "preventing" and "prevention" refers to the administration of therapy on a prophylactic or preventative basis to an individual who may ultimately manifest at least one symptom of a disease or condition (e.g., thrombosis) but who has not yet done so. Such individuals may be identified on the basis of risk factors that are known to correlate with the subsequent occurrence of the disease. Alternatively, prevention therapy may be administered without prior identification of a risk factor, as a prophylactic measure. Delaying the onset of the at least one symptom may also be considered prevention or prophylaxis.

As used herein the term "treat," "treating" or "treatment" refers to the administration of therapy to an individual who already manifests at least one symptom of a disease or condition (e.g., thrombosis), or who has previously manifested at least one symptom of a disease or condition.

As used herein, "acute illness" refers to any condition, illness, or disease that does or may cause severely restricted mobility, which is or may be characterized by a greater risk for thromboembolic complications than is present in the population generally. Nonlimiting examples include patients with cardiac insufficiency, respiratory failure, severe infections, and rheumatic diseases:

As used herein "enoxaparin sodium" refers to the low molecular weight heparin (LMWH) approved by the U.S. Food and Drug Administration (FDA) as Lovenox® (enoxaparin sodium injection) and any LMWH approved by the FDA pursuant to an application citing Lovenox® (enoxaparin sodium injection) as the listed drug. Enoxaparin sodium is available from Aventis and sold in the United States in the form of enoxaparin sodium injection, under the trademark Lovenox® (Clexane® in some other countries). In general, enoxaparin sodium is obtained by alkaline degradation of heparin benzyl ester derived from porcine intestinal mucosa. Its structure is characterized, for example, by a 2-0-sulfo-4-enepyranosuronic acid group at the nonreducing end and a 2-N,6-0-disulfo-D-glucosamine at the reducing end of the chain. The average molecular weight is about 4500 daltons. The molecular weight distribution is:
<2000 daltons ≤20%
2000 to 8000 daltons ≥68%
>8000 daltons ≤18%

Enoxaparin sodium injection is a sterile aqueous solution containing enoxaparin sodium. Enoxaparin sodium injection is available from Aventis at 100 mg/ml in prefilled syringes, graduated prefilled syringes, and multiple-dose vials. Enoxaparin sodium injection 100 mg/mL concentration contains 10 mg enoxaparin sodium (approximate anti-Factor Xa activity of 1000 IU [with reference to the W.H.O. First International Low Molecular Weight Heparin Reference Standard]) per 0.1 mL water for injection. Enoxaparin sodium injection is also available from Aventis at 150 mg/ml in graduated prefilled syringes. Enoxaparin sodium injection 150 mg/mL concentration contains 15 mg enoxaparin sodium (approximate anti-Factor Xa activity of 1500 IU [with reference to the W.H.O. First International Low Molecular Weight Heparin Reference Standard]) per 0.1 mL water for injection.

The enoxaparin sodium injection prefilled syringes and graduated prefilled syringes are preservative-free and intended for use only as a single-dose injection. The multiple-dose vial contains 15 mg/1.0 mL benzyl alcohol as a preservative. The pH of the injection is 5.5 to 7.5. Enoxaparin sodium injection may also be administered in an arterial line for a hemodialysis indication.

Because creatinine is found in stable plasma concentrations, is freely filtered and not reabsorbed, and is minimally secreted by the kidneys, creatinine clearance is used to estimate the glomerular filtration rate (GFR). The GFR in turn is the standard by which kidney function is assessed. The creatinine clearance test compares the level of creatinine in urine with the creatinine level in the blood. Clearance is often measured as milliliters/minute (mL/min). Creatinine clearance values may be calculated by the Cockroft-Gault formula. (Cockcroft and Gault, Nephron, Vol. 16, pp. 31-41 (1976)). In a normal human subject, creatinine clearance is > 80 mL/min. Renal impairment is a condition characterized by a creatinine clearance rate of ≤ 80 mL/min. Severe renal impairment is characterized by a creatinine clearance rate of < 30 mL/min.

Known enoxaparin sodium dosage regimens include 30 mg of enoxaparin sodium twice a day (approximately every 12 hours) for patients undergoing hip or knee replacement surgery, and 40 mg of enoxaparin sodium once a day (approximately every 24 hours) for patients undergoing abdominal surgery or medical patients during acute illness.

It has been found that in subjects with severe renal impairment the mean decrease in apparent plasma clearance was 39% and 50% in two studies. This was found to result in a 65% to 95% increase in AUC (area under the plasma activity-time curve to infinity) over one dosing interval at steady state. Given the prolongation of the elimination half-life in severe renal impairment, a dosage reduction sufficient to compensate for the increase in steady state AUC is desirable.

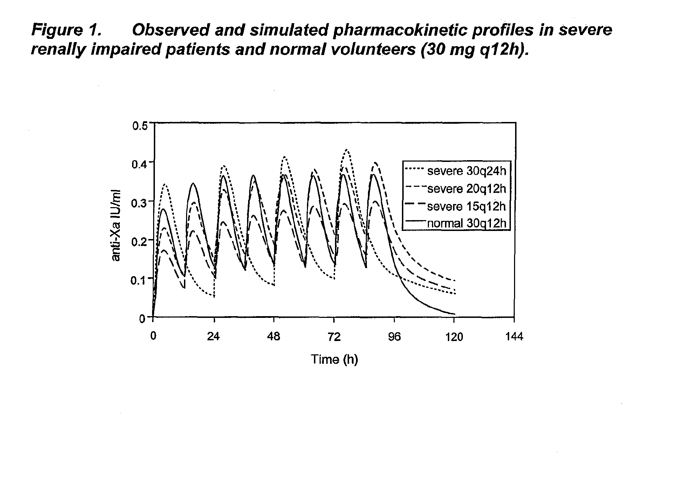

Using simulation curves (Figure 1 (Observed and simulated pharmacokinetic profiles in severely renally impaired patients and normal volunteers (30 mg q12h)).) and deduced pharmacokinetic parameters (Table 1) for dosages of 30 mg/12 h (30 milligrams/12 hours) in normal patients, and dosages of 15 mg/12 h, 20 mg/12 h, and 30 mg/24 h in patients with severe renal insufficiency, it has been predicted that adjustment of the 30 mg enoxaparin sodium twice daily dosage to 30 mg enoxaparin sodium once daily combines the advantage of adequate exposure and little accumulation and thus early achievement of steady-state. Using the simulation curves and pharmacokinetic parameters, 20 mg/12 h mimics the profile in normal subjects quite well, but because of the low dosing interval/half-life ratio, achievement of steady-state is expected to be longer and, especially for 15 mg/12 h, exposure at treatment initiation is expected to be low compared to the reference treatment. This is also explained by the relatively lower impact of severe renal impairment on exposure on day 1.

**Table 1.**

| Predicted pharmacokinetic parameters in severely renally impaired patients for alternative regimens to 30 mg q12h. | | | | | |
|---|---|---|---|---|---|
| | **Normal 30q12h** | **Severe 15q12h** | **Severe 20 q12h** | **Severe 30q24h** | |
| AUCτ1* | 2.30 | 1.50 | 2.00 | 4.04 | 2.02*** |
| **AUC τ8**** | **3.12** | **2.89** | **3.86** | **5.91** | **2.95***** |
| Amax τ1 | 0.344 | 0.221 | 0.295 | 0.342 | |
| **Amax τ8** | **0.367** | **0.298** | **0.398** | **0.429** | |
| Amin τ1 | 0.123 | 0.103 | 0.138 | 0.053 | |
| **Amin τ8** | **0.140** | **0.172** | **0.229** | **0.110** | |
| **Rac AUC τ8/** | **1.35** | **1.93** | | **1.46** | |
| **τ1** | **1.07** | **1.35** | | **1.26** | |
| Rac Amax | | | | | |
| Rac: accumulation ratio. | | | | | |

| | | | | | |
|---|---|---|---|---|---|
| * τ1: first dosing interval (0-12h, except severe 30q24h: 0-24h). | | | | | |
| ** τ8: 8th dosing interval (84-96h, except severe 30q24h: 4th interval 72-96h). | | | | | |
| *** Average AUC per 12h on day 1 and day 4, respectively. | | | | | |

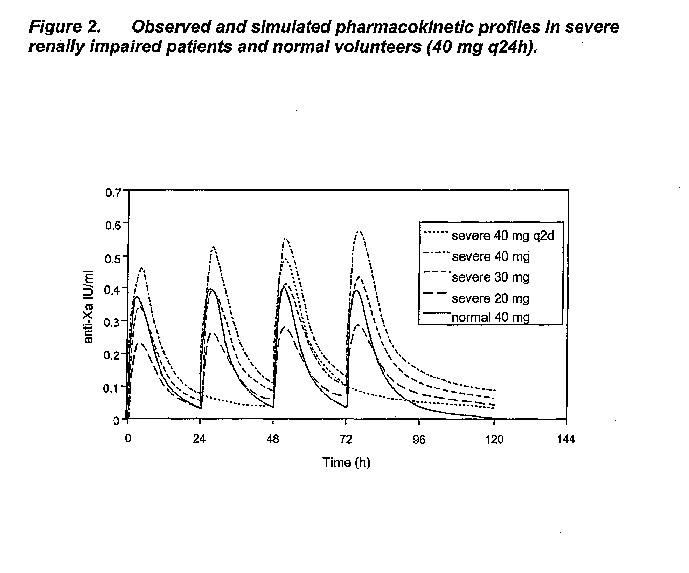

For the once-daily regimen of 40 mg, from simulation curves (Figure 2 (Observed and simulated pharmacokinetic profiles in severe renally impaired patients and normal volunteers (40 mg q24h)).) and deduced pharmacokinetic parameters (Table 2) for dosages of 40 mg/24 h in normal patients and, and dosages of 20 mg/24 h, 30 mg/24 h, and 40 mg/24 h in patients with sever renal insufficiency, it has been predicted that 30 mg enoxaparin sodium once daily appears desirable considering the whole treatment period; somewhat higher levels than in normal subjects would occur only in the elimination phase, but more adequate exposure than with a 20 mg dose is predicted to be obtained before reaching steady-state.

**Table 2.**

| Predicted pharmacokinetic parameters in severely renally impaired patients for alternative regimens to 40 mg q24h. | | | | | | |
|---|---|---|---|---|---|---|
| | Normal 40 mg qd | Severe 20 mg qd | Severe 30 mg qd | Severe 40 mg qd | Severe 40 mg q2d | |
| AUCτ1* | 3.87 | 2.70 | 4.06 | 5.41 | 6.62 | **3.31***** |
| **AUC τ4**** | **4.15** | **3.87** | **5.81** | **7.74** | **7.76** | **3.88***** |
| Amax τ1 | 0.371 | 0.228 | 0.342 | 0.457 | 0.457 | |
| **Amax τ4** | **0.394** | **0.287** | **0.430** | **0.574** | **0.491** | |
| Amin τ1 | 0.030 | 0.036 | 0.054 | 0.072 | 0.038 | |
| **Amin τ4** | **0.033** | **0.073** | **0.109** | **0.146** | **0.051** | |
| **Rac AUC** | **1.07** | **1.43** | | | **1.17** | |
| **Rac Amax** | **1.06** | **1.26** | | | **1.08** | |
| Rac: accumulation ratio. | | | | | | |

| | | | | | | |
|---|---|---|---|---|---|---|
| * τ1: first dosing interval (0-24h, except severe 40q2d: 0-48h). | | | | | | |
| ** τ4: 4th dosing interval (72-96h, except severe 40q2d: 2nd interval 48-96h). | | | | | | |
| *** Average AUC per day on days 1-2 and days 3-4, respectively. | | | | | | |

Given dose-linear exposure of enoxaparin anti-Xa activity, the same rule can apply to treatment doses, i.e., 1 mg/kg once daily as a substitute for both 1 mg/kg twice daily and 1.5 mg/kg once daily.

In an embodiment, the invention provides a method for the prevention of thrombotic episodes in a human patient with severe renal impairment comprising administering to said patient from more than 20mg to less than 40mg of enoxaparin sodium once daily. In another embodiment of the method, from 25mg to 35mg of enoxaparin sodium is administered once daily. In another embodiment of the method, about 30 mg of enoxaparin sodium is administered once daily. In another embodiment of the method, 30 mg of enoxaparin sodium is administered once daily. In a further embodiment, administration of the enoxaparin sodium begins prior to abdominal surgery, after hip or knee replacement surgery, or before hip replacement surgery. In another embodiment, the patient is a medical patient at risk for thromboembolic complications due to severely restricted mobility during acute illness. Administration of enoxaparin sodium is, for example, by injection, and further for example, by subcutaneous injection.

In another embodiment, the invention provides a method for the treatment of thrombotic episodes in a human with severe renal impairment comprising administering to said patient from more than 20mg to less than 40mg of a enoxaparin sodium once daily. In another embodiment of the method, from 25mg to 35mg of enoxaparin sodium is administered once daily. In yet another embodiment of the method, about 30 mg of enoxaparin sodium is administered once daily. In another embodiment of the method, 30 mg of enoxaparin sodium is administered once daily. In a further embodiment, administration of enoxaparin sodium begins prior to abdominal surgery, after hip or knee replacement surgery, or before hip replacement surgery. In another embodiment, the patient is a medical patient at risk for thromboembolic complications due to severely restricted mobility during acute illness. Administration of enoxaparin sodium is, for example, by injection and further for example by subcutaneous injection.

In another embodiment, the invention provides a method for the prevention of venous thrombosis in a postoperative human patient with severe renal impairment comprising administering to said patient from more than 20mg to less than 40mg of enoxaparin sodium once daily. In another embodiment of the method, from 25mg to 35mg of enoxaparin sodium is administered once daily. In another embodiment of the method, about 30 mg of enoxaparin sodium is administered once daily. In yet another embodiment of the method, 30 mg of enoxaparin sodium is administered once daily. In a further embodiment, administration of enoxaparin sodium begins prior to abdominal surgery, after hip or knee replacement surgery, or before hip replacement surgery. In another embodiment, the patient is a medical patient at risk for thromboembolic complications due to severely restricted mobility during acute illness. Administration of enoxaparin sodium is, for example, by injection and further for example by subcutaneous injection.

In another embodiment, the invention provides a therapeutic method for controlling thrombosis and/or decreasing blood hypercoagulation and/or hemorrhaging risks in a patient with severe renal impairment comprising administering to said patient from more than 20mg to less than 40mg of enoxaparin sodium once daily. In another embodiment of the method, from 25mg to 35mg of enoxaparin sodium is administered once daily. In another embodiment of the method, about 30 mg of enoxaparin sodium is administered once daily. In yet another embodiment of the method, 30 mg of enoxaparin sodium is administered once daily. In a further embodiment, administration of enoxaparin sodium begins prior to abdominal surgery, after hip or knee replacement surgery, or before hip replacement surgery. In another embodiment, the patient is a medical patient at risk for thromboembolic complications due to severely restricted mobility during acute illness. Administration of enoxaparin sodium is, for example, by injection and further for example by subcutaneous injection.

In providing enoxaparin sodium dosage regimens for patients with severe renal impairment, the dosage regimen for administration of enoxaparin sodium is based on the particular indication. For each indication, methods are provided herein that include methods for preventing and/or treating thrombotic episodes in a human patient, methods for preventing venous thrombosis in a postoperative human patient, and methods for controlling thrombosis and/or decreasing blood hypercoagulation and/or hemorrhaging risks in a patient.

In patients undergoing abdominal surgery who are at risk for thomboembolic complications, the initial dose of enoxaparin sodium is administered before abdominal surgery. Enoxaparin sodium is, for example, administered beginning approximately 2 hours prior to surgery. After the initial dose, the dosage can be administered once daily (approximately every 24 hours). Administration can be continued, for example, for approximately 7 to 10 days, and further for example, up to approximately 12 days. The dosage of enoxaparin sodium is, for example, from more than 20 mg to less than 40 mg, from 21 mg to 39 mg, from 22 mg to 38 mg, from 23 mg to 37 mg, from 24 mg to 36 mg, from 25 mg to 35 mg, from 26 mg to 34 mg, from 27 mg to 33 mg, from 28 mg to 32 mg, from 29 mg to 31 mg, about 30 mg, or 30 mg. Administration of enoxaparin sodium is, for example, by injection, and further for example, by subcutaneous injection.

An embodiment of the invention thus provides a method for the prevention of thrombotic episodes in a human patient with severe renal impairment comprising administering to said patient from more than 20mg to less than 40mg of enoxaparin sodium once daily beginning prior to abdominal surgery. In another embodiment, the method comprises administering to said patient from 25mg to 35mg of enoxaparin sodium once daily beginning prior to abdominal surgery. In another embodiment, the method comprises administering about 30 mg of enoxaparin sodium once daily beginning prior to abdominal surgery. In a further embodiment, the method comprises administering to said patient 30mg of enoxaparin sodium by subcutaneous injection once daily beginning prior to abdominal surgery.

An embodiment of the invention also provides a method for treatment of thrombotic episodes in a human patient with severe renal impairment comprising administering to said patient from more than 20mg to less than 40mg of enoxaparin sodium once daily beginning prior to abdominal surgery. In another embodiment, the method comprises administering to said patient from 25mg to 35mg of enoxaparin sodium once daily beginning prior to abdominal surgery. In yet another embodiment, the method comprises administering about 30 mg of enoxaparin sodium once daily beginning prior to abdominal surgery. In a further embodiment, the method comprises administering to said patient 30mg of enoxaparin sodium by subcutaneous injection once daily beginning prior to abdominal surgery.

An embodiment of the invention also provides a method for the prevention of venous thrombosis in a postoperative human patient with severe renal impairment comprising administering to said patient from more than 20mg to less than 40mg of enoxaparin sodium once daily beginning prior to abdominal surgery. In another embodiment, the method comprises administering to said patient from 25mg to 35mg of enoxaparin sodium once daily beginning prior to abdominal surgery. In yet another embodiment, the method comprises administering about 30 mg of enoxaparin sodium once daily beginning prior to abdominal surgery. In a further embodiment, the method comprises administering to said patient 30mg of enoxaparin sodium by subcutaneous injection once daily beginning prior to abdominal surgery.

An embodiment of the invention also provides a therapeutic method for controlling thrombosis and/or decreasing blood hypercoagulation and/or hemorrhaging risks in a human patient with severe renal impairment comprising administering to said patient from more than 20mg to less than 40mg of enoxaparin sodium once daily beginning prior to abdominal surgery. In another embodiment, the method comprises administering to said patient from 25mg to 35mg of enoxaparin sodium once daily beginning prior to abdominal surgery. In yet another embodiment, the method comprises administering about 30 mg of enoxaparin sodium once daily beginning prior to abdominal surgery. In a further embodiment, the method comprises administering to said patient 30mg of enoxaparin sodium by subcutaneous injection once daily.

In patients undergoing hip or knee replacement surgery, the initial dose of enoxaparin sodium can be administered approximately 12 to 24 hours after surgery, provided that hemostasis has been established. The dosage can be administered once daily (approximately every 24 hours), for example, for approximately 7 to 10 days, and further for example, for up to approximately 14 days. The dosage of enoxaparin sodium is, for example, from more than 20 mg to less than 40 mg, from 21 mg to 39 mg, from 22 mg to 38 mg, from 23 mg to 37 mg, from 24 mg to 36 mg, from 25 mg to 35 mg, from 26 mg to 34 mg, from 27 mg to 33 mg, from 28 mg to 32 mg, from 29 mg to 31 mg, about 30 mg, or 30 mg. Administration of enoxaparin sodium can be, for example, by injection, and further for example, by subcutaneous injection.

An embodiment of the invention provides a method for the prevention of thrombotic episodes in a human patient with severe renal impairment comprising administering to said patient from more than 20mg to less than 40mg of enoxaparin sodium once daily beginning after hip of knee replacement surgery. In another embodiment, the method comprises administering to said patient from 25mg to 35mg of enoxaparin sodium once daily beginning after hip of knee replacement surgery. In yet another embodiment, the method comprises administering about 30 mg of enoxaparin sodium once daily beginning after hip of knee replacement surgery. In a further embodiment, the method comprises administering to said patient 30mg of enoxaparin sodium by subcutaneous injection once daily beginning after hip of knee replacement surgery.

An embodiment of the invention also provides a method for treatment of thrombotic episodes in a human patient with severe renal impairment comprising administering to said patient from more than 20mg to less than 40mg of enoxaparin sodium once daily beginning after hip of knee replacement surgery. In another embodiment, the method comprises administering to said patient from 25mg to 35mg of enoxaparin sodium once daily beginning after hip of knee replacement surgery. In another embodiment, the method comprises administering about 30 mg of enoxaparin sodium once daily beginning after hip of knee replacement surgery. In a further embodiment, the method comprises administering to said patient 30mg of enoxaparin sodium by subcutaneous injection once daily beginning after hip of knee replacement surgery.

An embodiment of the invention also provides a method for the prevention of venous thrombosis in a postoperative human patient with severe renal impairment comprising administering to said patient from more than 20mg to less than 40mg of enoxaparin sodium once daily beginning after hip of knee replacement surgery. In another embodiment, the method comprises administering to said patient from 25mg to 35mg of enoxaparin sodium once daily beginning after hip of knee replacement surgery. In yet another embodiment, the method comprises administering about 30 mg of enoxaparin sodium once daily beginning after hip of knee replacement surgery. In a further embodiment, the method comprises administering to said patient 30mg of enoxaparin sodium by subcutaneous injection once daily beginning after hip of knee replacement surgery.

An embodiment of the invention also provides a therapeutic method for controlling thrombosis and/or decreasing blood hypercoagulation and/or hemorrhaging risks in a human patient with severe renal impairment comprising administering to said patient from more than 20mg to less than 40mg of enoxaparin sodium once daily beginning after hip of knee replacement surgery. In another embodiment, the method comprises administering to said patient from 25mg to 35mg of enoxaparin sodium once daily beginning after hip of knee replacement surgery. In another embodiment, the method comprises administering about 30 mg of enoxaparin sodium once daily beginning after hip of knee replacement surgery. In a further embodiment, the method comprises administering to said patient 30mg of enoxaparin sodium by subcutaneous injection once daily beginning after hip of knee replacement surgery.

For hip replacement surgery patients, a dose of enoxaparin sodium may be administered approximately 12 (± 3) hours prior to surgery. Following this initial phase of thromboprophylaxis, the dosage can be, for example, administered once daily (approximately every 24 hours) for approximately 3 weeks (approximately 21 days). The dosage of enoxaparin sodium is, for example, from more than 20 mg to less than 40 mg, from 21 mg to 39 mg, from 22 mg to 38 mg, from 23 mg to 37 mg, from 24 mg to 36 mg, from 25 mg to 35 mg, from 26 mg to 34 mg, from 27 mg to 33 mg, from 28 mg to 32 mg, from 29 mg to 31 mg, about 30 mg, or 30 mg. Administration of enoxaparin sodium can be, for example, by injection, and further for example, by subcutaneous injection.

An embodiment of the invention provides a method for the prevention of thrombotic episodes in a human patient with severe renal impairment comprising administering to said patient from more than 20mg to less than 40mg of enoxaparin sodium once daily beginning approximately 12 hours before hip replacement surgery. In another embodiment, the method comprises administering to said patient from 25mg to 35mg of enoxaparin sodium once daily beginning approximately 12 hours before hip replacement surgery. In another embodiment, the method comprises administering about 30 mg of enoxaparin sodium once daily beginning approximately 12 hours before hip replacement surgery. In a further embodiment, the method comprises administering to said patient 30mg of enoxaparin sodium by subcutaneous injection once daily beginning approximately 12 hours before hip replacement surgery.

An embodiment of the invention also provides a method for the prevention of thrombotic episodes in a human patient with severe renal impairment comprising administering to said patient from more than 20mg to less than 40mg of enoxaparin sodium once daily beginning approximately 12 hours before hip replacement surgery. In another embodiment, the method comprises administering to said patient from 25mg to 35mg of enoxaparin sodium once daily beginning approximately 12 hours before hip replacement surgery. In yet another embodiment, the method of the invention comprises administering about 30 mg of enoxaparin sodium once daily beginning approximately 12 hours before hip replacement surgery. In a further embodiment, the method comprises administering to said patient 30mg of enoxaparin sodium by subcutaneous injection once daily beginning approximately 12 hours before hip replacement surgery.

An embodiment of the invention also provides a method for the prevention of venous thrombosis in a postoperative human patient with severe renal impairment comprising administering to said patient from more than 20mg to less than 40mg of enoxaparin sodium once daily beginning approximately 12 hours before hip replacement surgery. In another embodiment, the method comprises administering to said patient from 25mg to 35mg of enoxaparin sodium once daily beginning approximately 12 hours before hip replacement surgery. In yet another embodiment, the method comprises administering about 30 mg of enoxaparin sodium once daily beginning approximately 12 hours before hip replacement surgery. In a further embodiment, the method comprises administering to said patient 30mg of enoxaparin sodium by subcutaneous injection once daily beginning approximately 12 hours before hip replacement surgery.

An embodiment of the invention also provides a therapeutic method for controlling thrombosis and/or decreasing blood hypercoagulation and/or hemorrhaging risks in a human patient with severe renal impairment comprising administering to said patient from more than 20mg to less than 40mg of enoxaparin sodium once daily beginning approximately 12 hours before hip replacement surgery. In another embodiment, the method comprises administering to said patient from 25mg to 35mg of enoxaparin sodium once daily beginning approximately 12 hours before hip replacement surgery. In yet another embodiment, the method comprises administering about 30 mg of enoxaparin sodium once daily beginning approximately 12 hours before hip replacement surgery. In a further embodiment, the method comprises administering to said patient 30mg of enoxaparin sodium by subcutaneous injection once daily beginning approximately 12 hours before hip replacement surgery.

For medical patients with severe renal impairment at risk for thromboembolic complications due to severely restricted mobility during acute illness, such as patients with cardiac insufficiency, respiratory failure, severe infections, and rheumatic diseases, enoxaparin sodium may be administered once daily. The dosage is, for example, administered once daily (approximately every 24 hours), such as, for approximately 6 to 11 days, and further for example, up to approximately 14 days. The dosage of enoxaparin sodium is, for example, from more than 20 mg to less than 40 mg, from 21 mg to 39 mg, from 22 mg to 38 mg, from 23 mg to 37 mg, from 24 mg to 36 mg, from 25 mg to 35 mg, from 26 mg to 34 mg, from 27 mg to 33 mg, from 28 mg to 32 mg, from 29 mg to 31 mg, about 30 mg, or 30 mg. Administration of enoxaparin sodium can be, for example, by injection, and further for example, by subcutaneous injection.

An embodiment of the invention provides a method for the prevention of thrombotic episodes in a human patient with severe renal impairment during acute illness comprising administering to said patient from more than 20mg to less than 40mg of enoxaparin sodium once daily. In another embodiment, the method comprises administering to said patient from 25mg to 35mg of enoxaparin sodium once daily. In yet another embodiment, the method comprises administering about 30 mg of enoxaparin sodium once daily. In a further embodiment, the method comprises administering to said patient 30mg of enoxaparin sodium by subcutaneous injection once daily.

Another embodiment of the invention also provides a method for the treatment of thrombotic episodes in a human patient with severe renal impairment during acute illness comprising administering to said patient from more than 20mg to less than 40mg of enoxaparin sodium once daily. In another embodiment, the method comprises administering to said patient from 25mg to 35mg of enoxaparin sodium once daily. In yet another embodiment, the method comprises administering about 30 mg of enoxaparin sodium once daily. In a further embodiment, the method comprises administering to said patient 30mg of enoxaparin sodium by subcutaneous injection once daily.

An embodiment of the invention also provides a method for the prevention of venous thrombosis in a human patient with severe renal impairment during acute illness comprising administering to said patient from more than 20mg to less than 40mg of enoxaparin sodium once daily. In another embodiment, the method comprises administering to said patient from 25 mg to 35 mg of enoxaparin sodium once daily. In yet another embodiment, the method comprises administering about 30 mg of enoxaparin sodium once daily. In a further embodiment, the method comprises administering to said patient 30mg of enoxaparin sodium by subcutaneous injection once daily.

An embodiment of the invention also provides a method for controlling thrombosis and/or decreasing blood hypercoagulation and/or hemorrhaging risks in a human patient with severe renal impairment during acute illness comprising administering to said patient from more than 20mg to less than 40mg of enoxaparin sodium once daily. In another embodiment, the method comprises administering to said patient from 25mg to 35mg of enoxaparin sodium once daily. In yet another embodiment, the method comprises administering about 30 mg of enoxaparin sodium once daily. In a further embodiment, the method comprises administering to said patient 30mg of enoxaparin sodium by subcutaneous injection once daily.

In patients suffering from unstable angina or non-Q-wave myocardial infarction a dose of enoxaparin sodium can be administered once daily. The dosage can be administered for a minimum of 2 days and continued until clinical stabilization. The dosage can be, for example, administered for 2 to 8 days, and further for example up to about 12.5 days. The dosage can be, for example, from more than 0.5 mg/kg to less than 1.5 mg/kg, from 0.6 mg/kg to 1.4 mg/kg, from 0.7 mg/kg to 1.3 mg/kg, from 0.8 mg/kg to 1.2 mg/kg, from 0.9 mg/kg to 1.1 mg/kg about 1 mg/kg, or 1 mg/kg. Kg refers to body weight of the patient in kilograms. Administration can, for example, be by injection, and further for example by subcutaneous injection. Administration of the enoxaparin sodium may be accompanied, for example, by concurrent once daily administration of approximately 100 to 325 mg of aspirin.

An embodiment of the invention provides a method for treatment of unstable Angina and non-Q-wave myocardial infarction in a patient with severe renal impairment comprising administering to said patient from more than 0.5 mg/kg body weight to less than 1.5 mg/kg body weight of enoxaparin sodium once daily. In an embodiment of the method, about 1 mg/kg body weight of enoxaparin sodium can be administered once daily. In a further embodiment of the method, 1 mg/kg body weight of enoxaparin sodium can be administered by subcutaneous injection once daily. In another embodiment of the method, approximately 100 to 325 mg of aspirin can also be administered concurrently once daily.

In outpatient treatment of patients with acute deep vein thrombosis without pulmonary embolism who can be treated at home, inpatient (hospital) treatment of patients with acute deep vein thrombosis with pulmonary embolism, or inpatient (hospital) treatment of patients with acute deep vein thrombosis without pulmonary embolism who are not candidates for outpatient treatment, a dose of enoxaparin sodium can be administered once daily. The dosage can be administered for a minimum of 5 days and until a therapeutic oral anticoagulant effect has been achieved (International Normalization Ratio 2.0 to 3.0). The dosage can be, for example, administered for about 7 days and may be administered up to about 17 days. The dosage can be, for example, from more than 0.5 mg/kg to less than 1.5 mg/kg, from 0.6 mg/kg to 1.4 mg/kg, from 0.7 mg/kg to 1.3 mg/kg, from 0.8 mg/kg to 1.2 mg/kg, from 0.9 mg/kg to 1.1 mg/kg about 1 mg/kg, or 1 mg/kg. Kg refers to the body weight of the patient in kilograms. Administration can be, for example, by injection, and further for example, by subcutaneous injection. Administration of the enoxaparin sodium can be done in conjunction with daily administration of warfarin sodium therapy initiated within about 72 hours of the initiation of enoxaparin sodium administration.

An embodiment of the invention provides a method for outpatient treatment of patients with severe renal impairment with acute deep vein thrombosis without pulmonary embolism who can be treated at home, inpatient (hospital) treatment of patients with severe renal impairment and with acute deep vein thrombosis with pulmonary embolism, or inpatient (hospital) treatment of patients with severe renal impairment and with acute deep vein thrombosis without pulmonary embolism who are not candidates for outpatient treatment, comprising administering to said patient from more than 0.5 mg/kg body weight to less than 1.5 mg/kg body weight of enoxaparin sodium once daily. In an embodiment of the method, about 1 mg/kg body weight of enoxaparin sodium is administered once daily. In a further embodiment of the method, 1 mg/kg body weight of enoxaparin sodium is administered by subcutaneous injection once daily. In another embodiment of the method, warfarin sodium therapy is initiated within about 72 hours of the initiation of enoxaparin sodium administration and provided in conjunction therewith.

Articles of manufacture for use in administering a dosage regimen of enoxaparin sodium to a patient with severe renal impairment are also provided. The article of manufacture comprises enoxaparin sodium and instructions for administering the enoxaparin sodium to the patient. The instructions are designed to achieve administration of a dosage regimen of enoxaparin sodium to the patient. The actual dosage regimen for administration is based on the particular indication.

Articles of manufacture for use in connection with the prevention or treatment of thrombotic episodes in a human patient with severe renal impairment by the methods of the invention comprise enoxaparin sodium and instructions (e.g., label, labeling, or package insert) for administering the enoxaparin sodium to the patient, said instructions being designed to achieve administration to said patient of a dosage of enoxaparin sodium once daily. The dosage of enoxaparin sodium is, for example, from more than 20 mg to less than 40 mg, from 21 mg to 39 mg, from 22 mg to 38 mg, from 23 mg to 37 mg, from 24 mg to 36 mg, from 25 mg to 35 mg, from 26 mg to 34 mg, from 27 mg to 33 mg, from 28 mg to 32 mg, or from 29 mg to 31 mg, about 30mg, or 30 mg. The enoxaparin sodium is, for example, contained within a vial, and further for example, contained within a prefilled syringe. The prefilled syringe and instructions are, for example, contained within a suitable package. The enoxaparin sodium can be, for example, administered by injection, and further for example, administered by subcutaneous injection.

An embodiment of the invention provides an article of manufacture for use in connection with the prevention of thrombotic episodes in a human patient with severe renal impairment comprising enoxaparin sodium and instructions designed to achieve administration to said patient of from more than 20mg to less than 40mg of the enoxaparin sodium once daily. In another embodiment, the article of manufacture comprises enoxaparin sodium and instructions designed to achieve administration to said patient of 25mg to 35mg of enoxaparin sodium once daily. In yet another embodiment the article of manufacture comprises enoxaparin sodium and instructions designed to achieve administration to said patient of about 30 mg of enoxaparin sodium once daily. In a further embodiment of the article of manufacture, the enoxaparin sodium is contained within a syringe and the instructions are designed to achieve administration to said patient of 30 mg of the enoxaparin sodium by subcutaneous injection once daily.

An embodiment of the invention provides an article of manufacture for use in connection with the treatment of thrombotic episodes in a human patient with severe renal impairment comprising enoxaparin sodium and instructions designed to achieve administration to said patient of from more than 20mg to less than 40mg of the enoxaparin sodium once daily. In another embodiment, the article of manufacture comprises enoxaparin sodium and instructions designed to achieve administration to said patient of 25mg to 35mg of enoxaparin sodium once daily. In yet another embodiment, the article of manufacture comprises enoxaparin sodium and instructions designed to achieve administration to said patient of about 30 mg of enoxaparin sodium once daily. In a further embodiment of the article of manufacture, the enoxaparin sodium is contained within a syringe and the instructions are designed to achieve administration to said patient of 30 mg of the enoxaparin sodium by subcutaneous injection once daily.

Articles of manufacture for use in connection with the treatment of unstable angina or non-Q-wave myocardial infarction in a human patient with severe renal impairment by the methods of the invention comprise enoxaparin sodium and instructions (e.g., label, labeling, or package insert) for the use thereof, said instructions being designed to achieve administration to said patient of a dosage of enoxaparin sodium once daily. The dosage is, for example, from more than 0.5 mg/kg to less than 1.5 mg/kg, from 0.6 mg/kg to 1.4 mg/kg, from 0.7 mg/kg to 1.3 mg/kg, from 0.8 mg/kg to 1.2 mg/kg, from 0.9 mg/kg to 1.1 mg/kg about 1 mg/kg, or 1 mg/kg. Kg refers to the body weight of the patient in kilograms. The enoxaparin sodium is, for example, contained within a vial, and further for example, contained within a prefilled syringe. The syringe and instructions for using the syringe, which delivers the desired dosage, are, for example, contained within a suitable package. The enoxaparin sodium is, for example, administered by injection, and further for example, administered by subcutaneous injection. Administration of the enoxaparin sodium may, for example, be accompanied by daily concurrent administration of approximately 100 to 325 mg of aspirin.

An embodiment of the invention provides an article of manufacture for use in connection with the treatment of unstable angina or non-Q-wave myocardial infarction in a human patient with severe renal impairment comprising enoxaparin sodium and instructions designed to achieve administration of from more than 0.5 mg/kg body weight to less than 1.5 mg/kg body weight of the enoxaparin sodium once daily. In another embodiment, the article of manufacture of the invention comprises enoxaparin sodium and instructions designed to achieve administration of about 1 mg/kg body weight of the enoxaparin sodium once daily. In a further embodiment of the article of manufacture, the enoxaparin sodium is contained within a prefilled syringe and the instructions direct the use of the syringe to achieve administration of 1 mg/kg body weight of the enoxaparin sodium to the patient by subcutaneous injection once daily. Administration of the enoxaparin sodium may, for example, be accompanied by daily concurrent administration of approximately 100 to 325 mg of aspirin.

Articles of manufacture for use in connection with the treatment of a patient with acute deep vein thrombosis without pulmonary embolism who can be treated at home, inpatient (hospital) treatment of patients with acute deep vein thrombosis with pulmonary embolism, or inpatient (hospital) treatment of patients with acute deep vein thrombosis without pulmonary embolism who are not candidates for outpatient treatment, comprise enoxaparin sodium and instructions (e.g., label, labeling, or package insert) for achieving administration of a dosage of enoxaparin sodium once daily. The dosage is, for example, from more than 0.5 mg/kg to less than 1.5 mg/kg, from 0.6 mg/kg to 1.4 mg/kg, from 0.7 mg/kg to 1.3 mg/kg, from 0.8 mg/kg to 1.2 mg/kg, from 0.9 mg/kg to 1.1 mg/kg about 1 mg/kg, or 1 mg/kg. Kg refers to the body weight of the patient in kilograms. The enoxaparin sodium is, for example, contained within a vial, and further for example, contained within a prefilled syringe. The prefilled syringe and instructions to achieve the desired administration, are, for example, contained within a suitable package. The enoxaparin sodium can be, for example, administered by injection, and further for example, administered by subcutaneous injection. Administration of the enoxaparin sodium may, for example, be accompanied by daily concurrent administration of warfarin sodium therapy initiated within about 72 hours of the initiation of enoxaparin sodium administration.

An embodiment of the invention provides an article of manufacture for use in connection with the treatment of patients with acute deep vein thrombosis without pulmonary embolism who can be treated at home, inpatient (hospital) treatment of patients with acute deep vein thrombosis with pulmonary embolism, or inpatient (hospital) treatment of patients with acute deep vein thrombosis without pulmonary embolism who are not candidates for outpatient treatment, in a human patient with severe renal impairment, comprising enoxaparin sodium and instructions for achieving administration of from more than 0.5 mg/kg body weight to less than 1.5 mg/kg body weight of the enoxaparin sodium once daily. In another embodiment, the article manufacture comprises enoxaparin sodium and instructions for achieving administration of about 1 mg/kg body weight of the enoxaparin sodium once daily. In a further embodiment of the article of manufacture, the enoxaparin sodium is contained within a prefilled syringe and instructions for use of the prefilled syringe, designed to achieve administration of 1 mg/kg body weight of the enoxaparin sodium to the patient by subcutaneous injection once daily. In another embodiment of the article of manufacture, administration of the enoxaparin sodium is, for example, accompanied by daily concurrent administration of warfarin sodium therapy initiated within about 72 hours of the initiation administration of the heterogenous admixture of sulfated heparinic polysaccharides.

It will be readily apparent to one of ordinary skill in the relevant arts that other suitable modifications and adaptations to the methods and applications described herein are suitable and may be made without departing from the scope of the invention or any embodiment thereof. Having now described the present invention in detail, the same will be more clearly understood by reference to the following examples of the invention, which are based on pharmacokinetic modeling and are included herewith for purposes of illustration only and are not intended to be limiting of the invention.

### EXAMPLES

### Example 1: Administration of Enoxaparin Sodium to Prevent or Treat Thrombotic Episodes in a Human Patient with Severe Renal Impairement

A patient having severe renal impairment can be identified by the creatinine clearance test, by comparing the level of creatinine in the patient's urine with the creatinine level in the patient's blood. Clearance is measured in mL/min, and creatinine clearance values calculated by the Cockroft-Gault formula. (Cockcroft and Gault, Nephron, Vol. 16, pp. 31-41 (1976)). Based on the results of the creatinine clearance test, patients with a creatinine clearance value < 30 mL/min are identified as having severe renal failure.

A sterile aqueous solution containing 30 mg of enoxaparin sodium is subcutaneously injected into the patient once daily.

### Example 2: Administration of Enoxaparin Sodium to Prevent or Treat Thrombotic Episodes in a Human Patient with Severe Renal Impairement Who is Undergoing Abdominal Surgery.

A patient who is undergoing abdominal surgery can be identified as having severe renal impairment by the creatinine clearance test, by comparing the level of creatinine in the patient's urine with the creatinine level in the patient's blood. Clearance is measured in mL/min, and creatinine clearance values calculated by the Cockroft-Gault formula. (Cockcroft and Gault, Nephron, Vol. 16, pp. 31-41 (1976)). Based on the results of the creatinine clearance test, patients with a creatinine clearance value < 30 mL/min are identified as having severe renal failure.

A sterile aqueous solution containing 30 mg of enoxaparin sodium is subcutaneously injected into the patient once daily, beginning approximately 2 hours prior to abdominal surgery. After the initial dose, the dosage is administered once daily (approximately every 24 hours) for approximately 7 to 10 days.

### Example 3: Administration of Enoxaparin Sodium to Prevent or Treat Thrombotic Episodes in a Human Patient with Severe Renal Impairement Who is Undergoing Hip or Knee Replacement Surgery.

A patient who is undergoing hip or knee replacement surgery can be identified as having severe renal impairment by the creatinine clearance test, by comparing the level of creatinine in the patient's urine with the creatinine level in the patient's blood. Clearance is measured in mL/min, and creatinine clearance values calculated by the Cockroft-Gault formula. (Cockcroft and Gault, Nephron, Vol. 16, pp. 31-41 (1976)). Based on the results of the creatinine clearance test, patients with a creatinine clearance value < 30 mL/min are identified as having severe renal failure.

A sterile aqueous solution containing 30 mg of enoxaparin sodium is subcutaneously injected into the patient once daily, beginning approximately 12 to 24 hours after surgery, provided that hemostasis has been established. The dosage is thereafter administered once daily (approximately every 24 hours) for approximately 7 to 10 days.

### Example 4: Administration of Enoxaparin Sodium to Prevent or Treat Thrombotic Episodes in a Human Patient with Severe Renal Impairement Who is Undergoing Hip Replacement Surgery.

A patient who is undergoing hip replacement surgery can be identified as having severe renal impairment by the creatinine clearance test, by comparing the level of creatinine in the patient's urine with the creatinine level in the patient's blood. Clearance is measured in mL/min, and creatinine clearance values calculated by the Cockroft-Gault formula. (Cockcroft and Gauft, Nephron, Vol. 16, pp. 31-41 (1976)). Based on the results of the creatinine clearance test, patients with a creatinine clearance value < 30 mL/min are identified as having severe renal failure.

A sterile aqueous solution containing 30 mg of enoxaparin sodium is subcutaneously injected into the patient once daily, beginning approximately 12 (± 3) hours prior to surgery. Following this initial phase of thromboprophylaxis, the dosage is administered once daily (approximately every 24 hours) for approximately 3 weeks (approximately 21 days).

### Example 5: Administration of Enoxaparin Sodium to Prevent or Treat Thrombotic Episodes in a Human Patient with Severe Renal Impairement During Acute Illness.

A patient with acute illness can be identified as having severe renal impairment by the creatinine clearance test, by comparing the level of creatinine in the patient's urine with the creatinine level in the patient's blood. Clearance is measured in mL/min, and creatinine clearance values calculated by the Cockroft-Gault formula. (Cockcroft and Gault, Nephron, Vol. 16, pp. 31-41 (1976)). Based on the results of the creatinine clearance test, patients with a creatinine clearance value < 30 mL/min are identified as having severe renal failure.

A sterile aqueous solution containing 30 mg of enoxaparin sodium is subcutaneously injected into the patient once daily (approximately every 24 hours) for approximately 6 to 11 days.

### Example 6: Administration of Enoxaparin Sodium to Treat Unstable Angina or Non-Q-wave Myocardial Infarction in a Human Patient with Severe Renal Impairment.

A patient with unstable angina or non-Q-wave myocardial infarction can be identified as having severe renal impairment by the creatinine clearance test, by comparing the level of creatinine in the patient's urine with the creatinine level in the patient's blood. Clearance is measured in mL/min, and creatinine clearance values calculated by the Cockroft-Gault formula. (Cockcroft and Gault, Nephron, Vol. 16, pp. 31-41 (1976)). Based on the results of the creatinine clearance test, patients with a creatinine clearance value < 30 mL/min are identified as having severe renal failure.

A sterile aqueous solution containing 1 mg/kg (of the patient's body weight) of enoxaparin sodium is subcutaneously injected into the patient once daily for a minimum of 2 days and continued until clinical stabilization (approximately 2 to 8 days).

### Example 7: Administration of Enoxaparin Sodium to Treat Acute Deep Vein Thrombosis With or Without Pulmonary Embolism in a Human Patient with Severe Renal Impairment.

A patient with acute deep vein thrombosis without pulmonary embolism who can be treated at home, a patient with acute deep vein thrombosis with pulmonary embolism who can be treated on an inpatient (hospital) basis, or a patient with acute deep vein thrombosis without pulmonary embolism who is not a candidate for outpatient treatment and is treated on an inpatient (hospital) basis, can be identified as having severe renal impairment by the creatinine clearance test, by comparing the level of creatinine in the patient's urine with the creatinine level in the patient's blood. Clearance is measured in mL/min, and creatinine clearance values calculated by the Cockroft-Gault formula. (Cockcroft and Gault, Nephron, Vol. 16, pp. 31-41 (1976)). Based on the results of the creatinine clearance test, patients with a creatinine clearance value < 30 mL/min are identified as having severe renal failure.

A sterile aqueous solution containing 1 mg/kg (of the patient's body weight) of enoxaparin sodium is subcutaneously injected into the patient once daily for 5 to about 7 days, until a therapeutic oral anticoagulant effect has been achieved (International Normalization Ratio 2.0 to 3.0).

While the administration of enoxaparin sodium to patients with severe renal failure has been described in connection with certain embodiments, it is not intended to limit the invention to the particular forms set forth, but on the contrary, it is intended to cover such alternatives, modifications and equivalents as may be included within the spirit and scope of the invention as defined by the following claims.

## Claims

1. A method for the prevention of thrombotic episodes in a postoperative human patient with severe renal impairment, comprising administering to said patient 30mg of enoxaparin sodium by subcutaneous injection once daily, beginning approximately two hours prior to abdominal surgery.

2. A method as recited in claim 2, wherein said administration is continued for approximately 7-10 days.

3. A method for the treatment of thrombotic episodes in a postoperative human patient with severe renal impairment, comprising administering to said patient 30mg of enoxaparin sodium by subcutaneous injection once daily, beginning approximately two hours prior to abdominal surgery.

4. A method as recited in claim 3, wherein said administration is continued for approximately 7-10 days.

5. A method for the prevention of venous thrombosis in a postoperative human patient with severe renal impairment, comprising administering to said patient 30mg of enoxaparin sodium by subcutaneous injection once daily, beginning approximately two hours prior to abdominal surgery.

6. A method as recited in claim 5, wherein said administration is continued for approximately 7-10 days.

7. A therapeutic method for controlling thrombosis and/or decreasing blood hypercoagulation and/or hemorrhaging risks in a postoperative human patient with severe renal impairment, comprising administering to said patient 30mg of enoxaparin sodium by subcutaneous injection once daily, beginning approximately two hours prior to abdominal surgery.

8. A method as recited in claim 7, wherein said administration is continued for approximately 7-10 days.

9. A method for the prevention of thrombotic episodes in a human patient with severe renal impairment, comprising administering to said patient 30mg enoxaparin sodium by subcutaneous injection once daily, beginning approximately 12 to 24 hours after hip or knee replacement surgery.

10. A method as recited in claim 9, wherein said administration is continued for approximately 7-10 days.

11. A method for the treatment of thrombotic episodes in a human patient with severe renal impairment, comprising administering to said patient 30mg of enoxaparin sodium by subcutaneous injection once daily, beginning approximately 12 to 24 hours after hip or knee replacement surgery.

12. A method as recited in claim 11, wherein said administration is continued for approximately 7-10 days.

13. A method for the prevention of venous thrombosis in a human patient with severe renal impairment, comprising administering to said patient 30mg of enoxaparin sodium by subcutaneous injection once daily, beginning approximately 12 to 24 hours after hip or knee replacement surgery.

14. A method as recited in claim 13, wherein said administration is continued for approximately 7-10 days.

15. A therapeutic method for controlling thrombosis and/or decreasing blood hypercoagulation and/or hemorrhaging risks in a postoperative human patient with severe renal impairment, comprising administering to said patient 30mg of enoxaparin sodium by subcutaneous injection once daily, beginning approximately 12 to 24 hours after hip or knee replacement surgery.

16. A method as recited in claim 15, wherein said administration is continued for approximately 7-10 days.

17. A method for the prevention of thrombotic episodes in a human patient with severe renal impairment, comprising administering to said patient 30mg of enoxaparin sodium by subcutaneous injection once daily, beginning 12 hours before hip replacement surgery.

18. A method as recited in claim 17, wherein said administration is continued for approximately three weeks.

19. A method for the treatment of thrombotic episodes in a human patient with severe renal impairment, comprising administering to said patient 30mg of enoxaparin sodium by subcutaneous injection once daily, beginning 12 hours before hip replacement surgery.

20. A method as recited in claim 19, wherein said administration is continued for approximately three weeks.

21. A method for the prevention of venous thrombosis in a postoperative human patient with severe renal impairment, comprising administering to said patient 30mg of enoxaparin sodium by subcutaneous injection once daily, beginning 12 hours before hip replacement surgery.

22. A method as recited in claim 21, wherein said administration is continued for approximately three weeks.

23. A therapeutic method for controlling thrombosis and/or decreasing blood hypercoagulation and/or hemorrhaging risks in a human patient with severe renal impairment, comprising administering to said patient 30mg of enoxaparin sodium by subcutaneous injection once daily, beginning 12 hours before hip replacement surgery.

24. A method as recited in claim 23, wherein said administration is continued for approximately three weeks.

25. A method for the prevention of thrombotic episodes in a human patient with severe renal impairment during acute illness, comprising administering to said patient 30mg of enoxaparin sodium by subcutaneous injection once daily.

26. A method as recited in claim 25, wherein said administration is continued for approximately 6 to 11 days.

27. A method for the treatment of thrombotic episodes in a human patient with severe renal impairment during acute illness, comprising administering to said patient 30mg of enoxaparin sodium by subcutaneous injection once daily.

28. A method as recited in claim 27, wherein said administration is continued for approximately 6 to 11 days.

29. A method for the prevention of venous thrombosis in a postoperative human patient with severe renal impairment during acute illness, comprising administering to said patient 30mg of enoxaparin sodium by subcutaneous injection once daily.

30. A method as recited in claim 29, wherein said administration is continued for approximately 6 to 11 days.

31. A therapeutic method for controlling thrombosis and/or decreasing blood hypercoagulation and/or hemorrhaging risks in a human patient with severe renal impairment during acute illness, comprising administering to said patient 30mg of enoxaparin sodium by subcutaneous injection once daily.

32. A method as recited in claim 31, wherein said administration is continued for approximately 6 to 11 days.

33. A method for treatment of unstable angina in a patient with severe renal impairment, comprising administering to said patient 1 mg/kg of a enoxaparin sodium by subcutaneous injection once daily.

34. A method as recited in claim 33 wherein said administration is continued for approximately 2 to 8 days.

35. A method for treatment of unstable angina in a patient with severe renal impairment, comprising administering to said patient 1 mg/kg of enoxaparin sodium by subcutaneous injection once daily, and concurrently administering approximately 100 to 325 mg of aspirin once daily.

36. A method as recited in claim 35 wherein said administration is continued for approximately 2 to 8 days.

37. A method for treatment of non-Q-wave myocardial infarction in a patient with severe renal impairment, comprising administering to said patient 1 mg/kg of enoxaparin sodium by subcutaneous injection once daily.

38. A method as recited in claim 37 wherein said administration is continued for approximately 2 to 8 days.

39. A method for treatment of non-Q-wave myocardial infarction in a patient with severe renal impairment, comprising administering to said patient 1 mg/kg of enoxaparin sodium by subcutaneous injection once daily, and concurrently administering approximately 100 to 325 mg of aspirin once daily.

40. A method as recited in claim 39 wherein said administration is continued for approximately 2 to 8 days.

41. A method for the prevention of thrombotic episodes in a human patient with severe renal impairment, comprising administering to said patient 30mg of enoxaparin sodium by subcutaneous injection once daily.

42. A method for the treatment of thrombotic episodes in a human patient with severe renal impairment, comprising administering to said patient 30mg of enoxaparin sodium by subcutaneous injection once daily.

43. A method for the prevention of venous thrombosis in a postoperative human patient with severe renal impairment, comprising administering to said patient 30mg of enoxaparin sodium by subcutaneous injection once daily.

44. A therapeutic method for controlling thrombosis and/or decreasing blood hypercoagulation and/or hemorrhaging risks in a patient with severe renal impairment, comprising administering to said patient 30mg of enoxaparin sodium by subcutaneous injection once daily.

45. An article of manufacture for use in connection with the prevention of thrombotic episodes in a human patient with severe renal impairment, comprising enoxaparin sodium contained within a syringe and instructions for the use of said enoxaprin sodium, said instructions being designed to achieve administration to said patient of 30 mg of said enoxaparin sodium by subcutaneous injection once daily.

46. An article of manufacture for use in connection with the treatment of thrombotic episodes in a human patient with severe renal impairment, comprising enoxaparin sodium contained within a syringe and instructions for the use of said enoxaprin sodium, said instructions being designed to achieve administration to said patient of 30 mg of said enoxaparin sodium by subcutaneous injection once daily.

47. An article of manufacture for use in connection with the treatment of unstable Angina in a human patient with severe renal impairment, comprising enoxaparin sodium contained within a syringe and instructions for the use of said enoxaprin sodium, said instructions being designed to achieve administration to said patient of 1 mg/kg body weight of said enoxaparin sodium by subcutaneous injection once daily.

48. A method for the prevention of thrombotic episodes in a human patient with severe renal impairment, comprising administering to said patient from more than 20mg to less than 40mg of enoxaparin sodium once daily.

49. A method for the prevention of thrombotic episodes in a human patient with severe renal impairment, comprising administering to said patient from 25mg to 35mg of enoxaparin sodium once daily.

50. A method for the prevention of thrombotic episodes in a human patient with severe renal impairment, comprising administering to said patient about 30mg of enoxaparin sodium once daily.

51. A method for the treatment of thrombotic episodes in a human with severe renal impairment, comprising administering to said patient from more than 20mg to less than 40mg of enoxaparin sodium once daily.

52. A method for the treatment of thrombotic episodes in a human with severe renal impairment, comprising administering to said patient from 25mg to 35mg of enoxaparin sodium once daily.

53. A method for the treatment of thrombotic episodes in a human with severe renal impairment, comprising administering to said patient about 30mg of enoxaparin sodium once daily.

54. A method for the prevention of venous thrombosis in a postoperative human patient with severe renal impairment, comprising administering to said patient from more than 20mg to less than 40mg of enoxaparin sodium once daily.

55. A method for the prevention of venous thrombosis in a postoperative human patient with severe renal impairment, comprising administering to said patient between 25mg and 35mg of enoxaparin sodium once daily.

56. A method for the prevention of venous thrombosis in a postoperative human patient with severe renal impairment, comprising administering to said patient about 30mg of enoxaparin sodium once daily.

57. A therapeutic method for controlling thrombosis and/or decreasing blood hypercoagulation and/or hemorrhaging risks in a patient with severe renal impairment, comprising administering to said patient from more than 20mg to less than 40mg of enoxaparin sodium once daily.

58. A therapeutic method for controlling thrombosis and/or decreasing blood hypercoagulation and/or hemorrhaging risks in a patient with severe renal impairment, comprising administering to said patient from 25mg to 35mg of enoxaparin sodium once daily.

59. A therapeutic method for controlling thrombosis and/or decreasing blood hypercoagulation and/or hemorrhaging risks in a patient with severe renal impairment, comprising administering to said patient about 30mg of enoxaparin sodium once daily.

60. A method for the prevention of thrombotic episodes in a postoperative human patient with severe renal impairment, comprising administering to said patient from more than 20mg to less than 40mg of enoxaparin sodium once daily beginning prior to abdominal surgery.

61. A method for the prevention of thrombotic episodes in a postoperative human patient with severe renal impairment, comprising administering to said patient from 25mg to 35mg of enoxaparin sodium once daily beginning prior to abdominal surgery.

62. A method for the prevention of thrombotic episodes in a postoperative human patient with severe renal impairment, comprising administering to said patient about 30mg of enoxaparin sodium once daily beginning prior to abdominal surgery.

63. A method for the treatment of thrombotic episodes in a postoperative human patient with severe renal impairment, comprising administering to said patient from more than 20mg to less than 40mg of enoxaparin sodium once daily beginning prior to abdominal surgery.

64. A method for the treatment of thrombotic episodes in a postoperative human patient with severe renal impairment, comprising administering to said patient from 25mg to 35mg of enoxaparin sodium once daily beginning prior to abdominal surgery.

65. A method for the treatment of thrombotic episodes in a postoperative human patient with severe renal impairment, comprising administering to said patient about 30mg of enoxaparin sodium once daily beginning prior to abdominal surgery.

66. A method for the prevention of venous thrombosis in a postoperative human patient with severe renal impairment, comprising administering to said patient from more than 20mg to less than 40mg of enoxaparin sodium once daily beginning prior to abdominal surgery.

67. A method for the prevention of venous thrombosis in a postoperative human patient with severe renal impairment, comprising administering to said patient from 25mg to 35mg of enoxaparin sodium once daily beginning prior to abdominal surgery.

68. A method for the prevention of venous thrombosis in a postoperative human patient with severe renal impairment, comprising administering to said patient about 30mg of enoxaparin sodium once daily beginning prior to abdominal surgery.

69. A therapeutic method for controlling thrombosis and/or decreasing blood hypercoagulation and/or hemorrhaging risks in a postoperative human patient with severe renal impairment, comprising administering to said patient from more than 20mg to less than 40mg of enoxaparin sodium once daily beginning prior to abdominal surgery.

70. A therapeutic method for controlling thrombosis and/or decreasing blood hypercoagulation and/or hemorrhaging risks in a postoperative human patient with severe renal impairment, comprising administering to said patient from 25mg to 35mg of enoxaparin sodium once daily beginning prior to abdominal surgery.

71. A therapeutic method for controlling thrombosis and/or decreasing blood hypercoagulation and/or hemorrhaging risks in a postoperative human patient with severe renal impairment, comprising administering to said patient about 30mg of enoxaparin sodium once daily beginning prior to abdominal surgery.

72. A method for the prevention of thrombotic episodes in a human patient with severe renal impairment, comprising administering to said patient from more than 20mg to less than 40mg of enoxaparin sodium once daily beginning after hip or knee replacement surgery.

73. A method for the prevention of thrombotic episodes in a human patient with severe renal impairment, comprising administering to said patient from 25mg to 35mg of enoxaparin sodium once daily beginning after hip or knee replacement surgery.

74. A method for the prevention of thrombotic episodes in a human patient with severe renal impairment, comprising administering to said patient about 30mg of enoxaparin sodium once daily beginning after hip or knee replacement surgery.

75. A method for the treatment of thrombotic episodes in a human patient with severe renal impairment, comprising administering to said patient from more than 20mg to less than 40mg of enoxaparin sodium once daily beginning after hip or knee replacement surgery.

76. A method for the treatment of thrombotic episodes in a human patient with severe renal impairment, comprising administering to said patient from 25mg to 35mg of enoxaparin sodium once daily beginning after hip or knee replacement surgery.

77. A method for the treatment of thrombotic episodes in a human patient with severe renal impairment, comprising administering to said patient about 30mg of enoxaparin sodium once daily beginning after hip or knee replacement surgery.

78. A method for the prevention of venous thrombosis in a postoperative human patient with severe renal impairment, comprising administering to said patient from more than 20mg to less than 40mg of enoxaparin sodium once daily beginning after hip or knee replacement surgery.

79. A method for the prevention of venous thrombosis in a postoperative human patient with severe renal impairment, comprising administering to said patient from 25mg to 35mg of enoxaparin sodium once daily beginning after hip or knee replacement surgery.

80. A method for the prevention of venous thrombosis in a postoperative human patient with severe renal impairment, comprising administering to said patient about 30mg of enoxaparin sodium once daily beginning after hip or knee replacement surgery.

81. A therapeutic method for controlling thrombosis and/or decreasing blood hypercoagulation and/or hemorrhaging risks in a human patient with severe renal impairment, comprising administering to said patient from more than 20mg to less than 40mg of enoxaparin sodium once daily beginning after hip or knee replacement surgery.

82. A therapeutic method for controlling thrombosis and/or decreasing blood hypercoagulation and/or hemorrhaging risks in a human patient with severe renal impairment, comprising administering to said patient from 25mg to 35mg of enoxaparin sodium once daily beginning after hip or knee replacement surgery.

83. A therapeutic method for controlling thrombosis and/or decreasing blood hypercoagulation and/or hemorrhaging risks in a human patient with severe renal impairment, comprising administering to said patient about 30mg of enoxaparin sodium once daily beginning after hip or knee replacement surgery.

84. A method for the prevention of thrombotic episodes in a human patient with severe renal impairment, comprising administering to said patient from more than 20mg to less than 40mg of enoxaparin sodium once daily beginning before hip replacement surgery.

85. A method for the prevention of thrombotic episodes in a human patient with severe renal impairment, comprising administering to said patient from 25mg to 35mg of enoxaparin sodium once daily beginning before hip replacement surgery.

86. A method for the prevention of thrombotic episodes in a human patient with severe renal impairment, comprising administering to said patient about 30mg of enoxaparin sodium once daily beginning before hip replacement surgery.

87. A method for the treatment of thrombotic episodes in a human patient with severe renal impairment, comprising administering to said patient from more than 20mg to less than 40mg of enoxaparin sodium once daily beginning before hip replacement surgery.

88. A method for the treatment of thrombotic episodes in a human patient with severe renal impairment, comprising administering to said patient from 25mg to 35mg of enoxaparin sodium once daily beginning before hip replacement surgery.

89. A method for the treatment of thrombotic episodes in a human patient with severe renal impairment, comprising administering to said patient about 30mg of enoxaparin sodium once daily beginning before hip replacement surgery.

90. A method for the prevention of venous thrombosis in a postoperative human patient with severe renal impairment, comprising administering to said patient from more than 20mg to less than 40mg of enoxaparin sodium once daily beginning before hip replacement surgery.

91. A method for the prevention of venous thrombosis in a postoperative human patient with severe renal impairment, comprising administering to said patient from 25mg to 35mg of enoxaparin sodium once daily beginning before hip replacement surgery.

92. A method for the prevention of venous thrombosis in a postoperative human patient with severe renal impairment, comprising administering to said patient about 30mg of enoxaparin sodium once daily beginning before hip replacement surgery.

93. A therapeutic method for controlling thrombosis and/or decreasing blood hypercoagulation and/or hemorrhaging risks in a human patient with severe renal impairment, comprising administering to said patient from more than 20mg to less than 40mg of enoxaparin sodium once daily beginning before hip replacement surgery.

94. A therapeutic method for controlling thrombosis and/or decreasing blood hypercoagulation and/or hemorrhaging risks in a human patient with severe renal impairment, comprising administering to said patient from 25mg to 35mg of enoxaparin sodium once daily beginning before hip replacement surgery.

95. A therapeutic method for controlling thrombosis and/or decreasing blood hypercoagulation and/or hemorrhaging risks in a human patient with severe renal impairment, comprising administering to said patient about 30mg of enoxaparin sodium once daily beginning before hip replacement surgery.

96. A method for the prevention of thrombotic episodes in a human patient with severe renal impairment during acute illness, comprising administering to said patient from more than 20mg to less than 40mg of enoxaparin sodium once daily.

97. A method for the prevention of thrombotic episodes in a human patient with severe renal impairment during acute illness, comprising administering to said patient from 25mg to 35mg of enoxaparin sodium once daily.

98. A method for the prevention of thrombotic episodes in a human patient with severe renal impairment during acute illness, comprising administering to said patient about 30mg of enoxaparin sodium once daily.

99. A method for the treatment of thrombotic episodes in a human with severe renal impairment during acute illness, comprising administering to said patient from more than 20mg to less than 40mg of enoxaparin sodium once daily.

100. A method for the treatment of thrombotic episodes in a human with severe renal impairment during acute illness, comprising administering to said patient from 25mg to 35mg of enoxaparin sodium once daily.

101. A method for the treatment of thrombotic episodes in a human with severe renal impairment during acute illness, comprising administering to said patient about 30mg of enoxaparin sodium once daily.

102. A method for the prevention of venous thrombosis in a postoperative human patient with severe renal impairment during acute illness, comprising administering to said patient from more than 20mg to less than 40mg of enoxaparin sodium once daily.

103. A method for the prevention of venous thrombosis in a postoperative human patient with severe renal impairment during acute illness, comprising administering to said patient from 25mg to 35mg of enoxaparin sodium once daily.

104. A method for the prevention of venous thrombosis in a postoperative human patient with severe renal impairment during acute illness, comprising administering to said patient about 30mg of enoxaparin sodium once daily.

105. A therapeutic method for controlling thrombosis and/or decreasing blood hypercoagulation and/or hemorrhaging risks in a patient with severe renal impairment during acute illness, comprising administering to said patient from more than 20mg to less than 40mg of enoxaparin sodium once daily.

106. A therapeutic method for controlling thrombosis and/or decreasing blood hypercoagulation and/or hemorrhaging risks in a patient with severe renal impairment during acute illness, comprising administering to said patient from 25mg to 35mg of enoxaparin sodium once daily.

107. A therapeutic method for controlling thrombosis and/or decreasing blood hypercoagulation and/or hemorrhaging risks in a patient with severe renal impairment during acute illness, comprising administering to said patient about 30mg of enoxaparin sodium once daily.

108. A method for treatment of unstable angina in a patient with severe renal impairment, comprising administering to said patient from more than 0.5 mg/kg to less than 1.5 mg/kg of enoxaparin sodium once daily.

109. A method for treatment of unstable angina in a patient with severe renal impairment, comprising administering to said patient about 1 mg/kg of enoxaparin sodium once daily.

110. A method for treatment of unstable angina in a patient with severe renal impairment, comprising administering to said patient from more than 0.5 to less than 1.5 mg/kg of enoxaparin sodium once daily, and concurrently administering approximately 100 to 325 mg of aspirin once daily.

111. A method for treatment of unstable angina in a patient with severe renal impairment, comprising administering to said patient about 1 mg/kg of enoxaparin sodium once daily, and concurrently administering approximately 100 to 325 mg of aspirin once daily.

112. A method for treatment of non-Q-wave myocardial infarction in a patient with severe renal impairment, comprising administering to said patient from more than 0.5 mg/kg to less than 1.5 mg/kg of enoxaparin sodium once daily.

113. A method for treatment of non-Q-wave myocardial infarction in a patient with severe renal impairment, comprising administering to said patient about 1 mg/kg of enoxaparin sodium once daily.

114. A method for treatment of non-Q-wave myocardial infarction in a patient with severe renal impairment, comprising administering to said patient from more than 0.5 mg/kg to less than 1.5 mg/kg of enoxaparin sodium once daily, and concurrently administering approximately 100 to 325 mg of aspirin once daily.

115. A method for treatment of non-Q-wave myocardial infarction in a patient with severe renal impairment, comprising administering to said patient about 1 mg/kg of enoxaparin sodium once daily, and concurrently administering approximately 100 to 325 mg of aspirin once daily.

116. An article of manufacture for use in connection with the prevention of thrombotic episodes in a human patient with severe renal impairment, comprising enoxaparin sodium and instructions for the use of said enoxaprin sodium, said instructions being designed to achieve administration to said patient of from more than 20mg to less than 40mg of enoxaparin sodium once daily.

117. An article of manufacture as recited in claim 116, wherein said enoxaparin sodium is contained within a syringe.

118. An article of manufacture as recited in claim 116, wherein said enoxaparin sodium is contained within a vial.

119. An article of manufacture as recited in claim 116, wherein said administration is by subcutaneous injection.

120. An article of manufacture for use in connection with the prevention of thrombotic episodes in a human patient with severe renal impairment, comprising enoxaparin sodium and instructions for the use of said enoxaprin sodium, said instructions being designed to achieve administration to said patient of 30mg of enoxaparin sodium once daily, wherein said enoxaparin sodium and said instructions are contained within a package.

121. An article of manufacture for use in connection with the treatment of thrombotic episodes in a human patient with severe renal impairment, comprising enoxaparin sodium and instructions for the use thereof, said instructions being designed to achieve administration to said patient of from more than 20mg to less than 40mg of enoxaparin sodium once daily.

122. An article of manufacture as recited in claim 121, wherein said enoxaparin sodium is contained within a syringe.

123. An article of manufacture as recited in claim 121, wherein said enoxaparin sodium is contained within a vial.

124. An article of manufacture as recited in claim 121, wherein said administration is by subcutaneous injection.

125. An article of manufacture for use in connection with the treatment of thrombotic episodes in a human patient with severe renal impairment, comprising enoxaparin sodium and instructions for the use of said enoxaprin sodium, said instructions being designed to achieve administration to said patient of 30mg of enoxaparin sodium once daily, wherein said enoxaparin sodium and said instructions are contained within a package.

126. An article of manufacture for use in connection with the treatment of unstable Angina in a human patient with severe renal impairment, comprising enoxaparin sodium and instructions for the use of said enoxaprin sodium, said instructions being designed to achieve administration to said patient of from more than 0.5 mg/kg to less than 1.5 mg/kg of enoxaparin sodium once daily.

127. An article of manufacture as recited in claim 126, wherein said enoxaparin sodium is contained within a syringe.

128. An article of manufacture as recited in claim 126, wherein said enoxaparin sodium is contained within a vial.

129. An article of manufacture as recited in claim 126, wherein said administration is by subcutaneous injection.

130. An article of manufacture for use in connection with the treatment of unstable Angina in a human patient with severe renal impairment, comprising enoxaparin sodium and instructions for the use of said enoxaprin sodium, said instructions being designed to achieve administration to said patient of 30mg of enoxaparin sodium once daily, wherein said enoxaparin sodium and said instructions are contained within a package.

131. An article of manufacture for use in connection with the prevention of venous thrombosis in a postoperative human patient with severe renal impairment, comprising enoxaparin sodium contained within a syringe and instructions for the use of said enoxaprin sodium, said instructions being designed to achieve administration to said patient of 30 mg of said enoxaparin sodium by subcutaneous injection once daily.

132. An article of manufacture for use in connection with the prevention of venous thrombosis in a postoperative human patient with severe renal impairment, comprising enoxaparin sodium and instructions for the use of said enoxaprin sodium, said instructions being designed to achieve administration to said patient of from more than 20mg to less than 40mg of enoxaparin sodium once daily.

133. An article of manufacture as recited in claim 132, wherein said enoxaparin sodium is contained within a syringe.

134. An article of manufacture as recited in claim 132, wherein said enoxaparin sodium is contained within a vial.

135. An article of manufacture as recited in claim 132, wherein said administration is by subcutaneous injection.

136. An article of manufacture for use in connection with the prevention of venous thrombosis in a postoperative human patient with severe renal impairment, comprising enoxaparin sodium and instructions for the use of said enoxaprin sodium, said instructions being designed to achieve administration to said patient of 30mg of enoxaparin sodium once daily, wherein said enoxaparin sodium and said instructions are contained within a package.

137. An article of manufacture for use in connection with controlling thrombosis and/or decreasing blood hypercoagulation and/or hemorrhaging risks in a human patient with severe renal impairment, comprising enoxaparin sodium contained within a syringe and instructions for the use of said enoxaprin sodium, said instructions being designed to achieve administration to said patient of 30 mg of said enoxaparin sodium by subcutaneous injection once daily.

138. An article of manufacture for use in connection with controlling thrombosis and/or decreasing blood hypercoagulation and/or hemorrhaging risks in a human patient with severe renal impairment, comprising enoxaparin sodium and instructions for the use of said enoxaprin sodium, said instructions being designed to achieve administration to said patient of from more than 20mg to less than 40mg of enoxaparin sodium once daily.

139. An article of manufacture as recited in claim 138, wherein said enoxaparin sodium is contained within a syringe.

140. An article of manufacture as recited in claim 138, wherein said enoxaparin sodium is contained within a vial.

141. An article of manufacture as recited in claim 138, wherein said administration is by subcutaneous injection.

142. An article of manufacture for use in connection with controlling thrombosis and/or decreasing blood hypercoagulation and/or hemorrhaging risks in a human patient with severe renal impairment, comprising enoxaparin sodium and instructions for the use of said enoxaprin sodium, said instructions being designed to achieve administration to said patient of 30mg of enoxaparin sodium once daily, wherein said enoxaparin sodium and said instructions are contained within a package.

143. An article of manufacture for use in connection with the treatment of non-Q-wave myocardial infarction in a human patient with severe renal impairment, comprising enoxaparin sodium contained within a syringe and instructions for the use of said enoxaprin sodium, said instructions being designed to achieve administration to said patient of 1 mg/kg body weight of said enoxaparin sodium by subcutaneous injection once daily.

144. An article of manufacture for use in connection with the treatment of non-Q-wave myocardial infarction in a human patient with severe renal impairment, comprising enoxaparin sodium and instructions for the use of said enoxaprin sodium, said instructions being designed to achieve administration to said patient of from more than 0.5 mg/kg to less than 1.5 mg/kg of enoxaparin sodium once daily.

145. An article of manufacture as recited in claim 144, wherein said enoxaparin sodium is contained within a syringe.

146. An article of manufacture as recited in claim 144, wherein said enoxaparin sodium is contained within a vial.

147. An article of manufacture as recited in claim 144, wherein said administration is by subcutaneous injection.

148. An article of manufacture for use in connection with the treatment of non-Q-wave myocardial infarction in a human patient with severe renal impairment, comprising enoxaparin sodium and instructions for the use of said enoxaprin sodium, said instructions being designed to achieve administration to said patient of 30mg of enoxaparin sodium once daily, wherein said enoxaparin sodium and said instructions are contained within a package.
